# EUROPEAN PATENT APPLICATION

(11) **EP 4 166 536 A1**
(43) Date of publication of application: **19.04.2023**
(21) Application number: 21306433.0
(22) Date of filing: 13.10.2021
(51) Int. Cl.: C07C 241/00, C07C 243/02, C06B 25/34

(54) **PROCESS FOR SYNTHESIZING BIS(2-NITRATOETHYL) DINITROXAMIDE**

(71) Applicant: Eurenco, 84700 Sorgues (FR)
(72) Inventor: Klapötke, Thomas, 81377 Munich (DE); Krumm, Burkhard, 81377 Munich (DE); Riedelsheimer, Christian, 81377 Munich (DE)
(74) Representative: Cabinet Beau de Loménie

(57) **Abstract**

The invention relates to a process for synthesizing bis(2-nitratoethyl)dinitroxamide which comprises reacting bis-hydroxyethyloxamide with nitric acid in the presence of an oleum solution. The invention also relates to a process for preparing an energetic composition from the thus synthesized bis(2-nitratoethyl)dinitroxamide

## Description

### Field of the invention

The present invention is in the field of energetic materials, and more specifically relates to a process for the synthesis of Bis(2-nitratoethyl)dinitroxamide.

### Background of the invention

In the last decades of energetic materials research, the search for environmental benign alternatives for commonly used compounds is one of the main objectives [1]. Heavy metal-free primary explosives and pyrotechnics, non-toxic secondary explosives and halogen-free oxidizers are still under investigation. "Green" high energy dense oxidizers (HEDOs) are generally developed to replace ammonium perchlorate (AP), which is employed as standard oxidizer in composite propellant mixtures for rocket and missile engines [1]. Ammonium perchlorate has numerous advantages such as its outstanding low price, great availability and favourable oxygen balance [1]. Nonetheless the perchlorate ion is a hazardous substance and is registered under the REACH Regulation [2]. Additionally upon combustion, large amounts of chlorinated products are formed and released in the atmosphere. These exhaust gases directly harm the environment and can be detected easily, leading to tactical disadvantages [1]. Several potential halogen-free alternatives have been synthesized over the last decades. Ionic compounds such as ammonium dinitramide and ammonium nitrate appear to be potent replacements [3-4]. Covalent structures generally utilize specific oxygen-rich moieties. Some promising alternatives are based on the trinitroethyl moiety, such as trinitroethyl nitrocarbamate, bis(trinitroethyl)oxalate and 2,2,2-trinitroethyl formate [5-7].

Bis(2-nitratoethyl)dinitroxamide is another energetic material. It was first described in DE 543 174. A method for preparing this compound is described by Stuart et al. [8]; the method comprises reacting dibutyl oxalate with monoethanolamine at about 100°C, and then nitrating the bis-hydroxyethyloxamide thus obtained with a mixed acid consisting of nitric acid and sulfuric acid in a ratio of about 60:40. Another method for preparing this compound is described by Kuchurov et al. [9]; the method comprises nitrating *N,N'*-bis(2-hydroxyethyl)oxalamide using nitrogen pentoxide in the presence of supercritic CO₂ at 0°C for 120 min under a pressure of 80 bar.

The present invention aims at providing a synthesis of Bis(2-nitratoethyl)dinitroxamide which is easy to implement, safe (no use of nitrogen pentoxide is required), devoid of CMR (carcinogenic, mutagenic, or toxic for reproduction) substances, and which can be scaled-up, and where the product obtained displays properties comparable or superior to those of trinitrotoluene (TNT).

### Summary of the invention

It has been found that bis(2-nitratoethyl)dinitroxamide can, under certain operating conditions, be prepared in a one-step process, as opposed to the conventional two-step process known in the art. This proves to be advantageous notably in terms of safety, cost (fewer reagents need to be used and then recycled), and suitability for large-scale implementation.

The invention thus relates to a process for synthesizing bis(2-nitratoethyl)dinitroxamide which comprises reacting bis-hydroxyethyloxamide with nitric acid in the presence of an oleum solution

The invention also relates to a process for preparing an energetic composition which comprises admixing bis(2-nitratoethyl)dinitroxamide as obtained by the above-mentioned synthesis with at least one compound selected from one of an energetic material, an additive and a metal.

### Description of the figures

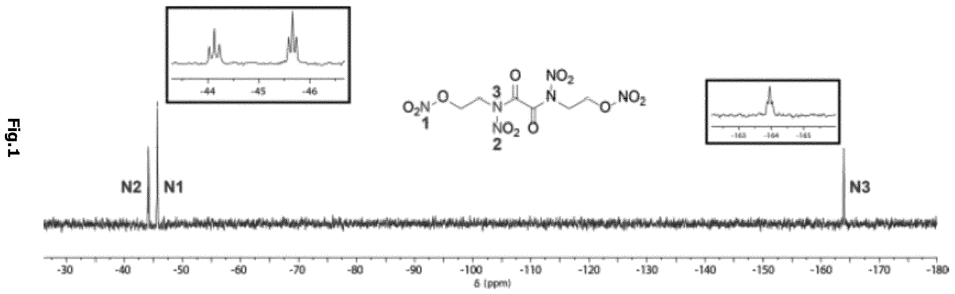

Figure 1 represents the ¹⁵N NMR spectrum of bis(2-nitratoethyl)dinitroxamide in [D₆]acetone.
Figure 2 represents the crystal structure of bis(2-nitratoethyl)dinitroxamide.

### Detailed description of the invention

A first aspect of the invention relates to a process for synthesizing bis(2-nitratoethyl)dinitroxamide which comprises reacting bis-hydroxyethyloxamide with nitric acid in the presence of an oleum solution.

Oleum, also known as fuming sulfuric acid, is a thick fuming yellow liquid resulting from the addition of sulfur trioxide to sulfuric acid. Oleums are generally represented by the formula H₂SO₄•(SO₃)ₓ or H₂O•(SO₃)_{y} where x and y independently represent the amount of sulfur trioxide added to sulfuric acid and dissolved in water. The formula where x=1 or y=2 is that of disulfuric acid (H₂S₂O₇) which is in equilibrium with sulfur trioxide and sulfuric acid in the oleum. The content of SO₃ in the oleum is expressed as the percentage of added SO₃ or the percentage of equivalent H₂SO₄ is the necessary amount of water was added. Usual concentrations of oleum solutions are in the range from about 20% (corresponding to 104.5% H₂SO₄) to about 65% (corresponding to 114.6% H₂SO₄). Pure H₂S₂O₇ is a solid having a melting point of 35°C.

In some embodiments the oleum solution used is a 20% to 65% oleum solution. In some embodiments, the oleum solution used is a 20% to 40% oleum solution, such as for example a 25% or 30% oleum solution.

In some embodiments, nitric acid and the oleum solution are used in a stoichiometric (equivalent) ratio in the range from about 5:1 (i.e. 5 equivalents of nitric acid for 1 equivalent of oleum solution) to about 15:1 (i.e. 15 equivalents of nitric acid for 1 equivalent of oleum solution). In some embodiments, said ratio is in the range from about 8:1 to about 12:1, such as for examples 9:1, 10:1 or 11:1.

In some embodiments, the nitric acid used is fuming nitric acid (i.e. the concentration of nitric acid is ≥ 86%). In some embodiments, the concentration of nitric acid is > 98%. In some embodiments, the nitration of bis-hydroxyethyloxamide is carried out at a temperature in the range from about -5°C to about +10°C, such as a temperature in the range from about -5°C to about +5°C.

In some embodiments, the nitration of bis-hydroxyethyloxamide is carried out for a period in the range from about 30 min to about 4h, such as for example a period in the range from about 30 min to about 2h.

The reaction product of bis-hydroxyethyloxamide, nitric acid and the oleum is recovered by conventional steps, such as extraction, filtration, drying and/or solvent removal.

In some embodiments, the bis-hydroxyethyloxamide is obtained by reacting diethyl oxalate with ethanolamine.

In some embodiments, the reaction of diethyl oxalate and ethanolamine is carried out in the presence of a nonpolar solvent, such as for example toluene.

Bis(2-nitratoethyl)dinitroxamide obtained as described above displays properties which make it suitable for use as an energetic material. The physical and energetic properties of this compound were determined as indicated in the section "Materials and methods", and are summarized in table 1.

**Table 1**

| Formula | C₆H₈N₆O₁₂ |
|---|---|
| MW [g mol⁻¹] | 356.16 |
| Tₘₑₗₜ [°C] | 78 |
| T_{dec} [°C] | 168 |
| IS [J] | 10 |
| FS [N] | 240 |
| ρ [g cm⁻³] | 1.72 |
| O [%] | 53.9 |
| Ω_{CO} [%] | 9.0 |
| Δ_{f}Hₘ° [kJ mol⁻¹] | -482.8 |
| | |
| V_{det} [m s⁻¹] ^{[g]} | 8017 |
| P_{det} [kbar] | 278 |
| Iₛₚ [s] | 250 |
| Iₛₚ [s] (15% Al) | 262 |
| Iₛₚ [s] (15% Al, 14% binder) | 235 |

| | |
|---|---|
| IS: Impact sensitivity / FS: Friction sensitivity / Tmelt: melting temperature /Tdec: decomposition temperature / p: density / Vdet: detonation velocity / Pdet: detonation pressure / O: oxygen content / Ω_{CO}: oxygen balance / Iₛₚ: specific impulse | |

It can be seen from table 1 that bis(2-nitratoethyl)dinitroxamide has a melting temperature (78°C) which is comparable to that of TNT (80°C) and has a detonation velocity (8017 m.s⁻¹) which is higher than that of TNT (6900 m.s⁻¹). It also has a higher density than TNT (1.72 vs 1.65 g.cm⁻³) and reaches a calculated specific impulse of *I*ₛₚ = 250s for the net compound and *I*ₛₚ = 262s in admixture with aluminum and *I*ₛₚ = 235s in admixture with aluminum and a binder (6% polybutadiene acrylic acid, 6% polybutadiene acrylonitrile and 2% bisphenol A ether).

These properties make it possible to use bis(2-nitratoethyl)dinitroxamide as an energetic material, in particular as an alternative to TNT.

Thus according to another aspect the invention relates to an energetic composition obtainable by (i) preparing bis(2-nitratoethyl)dinitroxamide by the process described above and (ii) admixing the prepared compound with at least one compound selected from an energetic material (other than bis(2-nitratoethyl)dinitroxamide), an additive and a metal.

In some embodiments the total amount of energetic material(s) in the energetic composition (i.e. bis(2-nitratoethyl)dinitroxamide and optionally one or more additional energetic materials) is at least 70 wt%, and up to 95 wt%, such as for example 70 wt%, 75 wt%, 80 wt%, 85 wt%, 90 wt% or 95 wt%, based on the weight of the energetic composition.

Examples of energetic materials which can be present in the energetic composition in addition to bis(2-nitratoethyl)dinitroxamide include: trinitrotoluene (TNT), hexogen (RDX), octogen (HMX), hexanitrohexaazaisowurtzitane (CL20), nitroguanidine (NGU), ethylene dinitramine (EDNA), N-guanylurea dinitramide (FOX 12 (GUDN)), 1,1-diamino-2,2-dinitroethylene (FOX 7 (DADE)), 5,5'-azotetrazolate of bis(triaminoguanidinium) (TAGZT), 5,5' azotetrazolate of dihydrazinium (DHDZT), 5,5'-bis(tetrazolyl) hydrazine (HBT), bis(2,2-dinitropropyl)nitramine (BDNPN), a nitropyrazole such as for example 3,4,5-trinitropyrazole (3,4,5-TNP), dihydroxylammonium 5-5'-bistetrazole-1,1'-diolate (TKX-50), and mixtures thereof.

In some embodiments, bis(2-nitratoethyl)dinitroxamide is admixed with at least one other energetic material, and the weight ratio between bis(2-nitratoethyl)dinitroxamide and said at least one other energetic material is in the range from about 5:95 to about 95:5, such as for example from about 20:80 to about 80:20, from about 30:70 to about 70:30, or from about 40:60 to about 60:40.

In some embodiments the energetic composition comprises an additive. In some embodiments the additive includes a processing agent, a plasticizer, an anti-cracking agent and mixtures thereof. In some embodiments the additive represents up to 10 wt%, for example up to 5 wt%, of the weight of the energetic composition.

In some embodiments the energetic composition comprises a metal such as, for example, Al or Mg. In some embodiments the metal represents up to 30 wt%, for example up to 20 wt%, up to 10 wt% or up to 5 wt%, of the weight of the energetic composition.

The energetic composition defined above can be used in various applications, for example as a propellant or as an explosive.

The invention will be better understood in the light of the examples which are given by way of illustration.

### Examples

### Materials and methods

Solvents were dried and purified with standard methods. Diethyl oxalate and ethanolamine are commercially available and were used without further purification. Raman spectra were recorded in glass tube with a Bruker MultiRAM FT-Raman spectrometer with a Klaastech DENICAFC LC-3/40 laser (Nd:YAG, 1064 nm, up to 1000 mW) in the range of 4000-400 cm⁻¹. Relative intensity is given in percent. IR spectra were recorded with a Perkin-Elmer Spectrum BX-FTIR spectrometer coupled with a Smiths ATR DuraSample IRII device. Measurements were recorded in the range of 4000-650 cm⁻¹. All Raman and IR spectra were measured at ambient temperature. NMR spectra were recorded with JEOL Eclipse and Bruker TR 400 MHz spectrometers at 25 °C. Chemical shifts were determined with respect to external standards Me₄Si (¹H, 399.8 MHz); (¹³C, 100.5 MHz); MeNO₂ (¹⁴N/¹⁵N, 28.9/40.7 MHz). Elemental analyses (EA) were obtained with a Vario EL Elemental Analyzer.

The sensitivity data were acquired by measurements with a BAM drop hammer and a BAM friction tester (BAM stands for "Bundesanstalt für Materialforschung und -prüfung"). Melting and decomposition points were determined by differential thermal calorimetry (DTA) using a OZResearch DTA 552-Ex instrument at a heating rate of 5°C/min. Measurements were performed in open glass vessels against a reference material up to 400°C.

The crystal structure data were obtained using an Oxford Xcalibur CCD Diffraktometer with a KappaCCD detector at low temperature (173 K, 123 K). Mο-Kα radiation (λ = 0.71073 Å) was delivered by a Spellman generator (voltage 50 kV, current 40 mA). Data collection and reduction were performed using the CRYSALIS CCD [10] and CRYSALIS RED [11] software, respectively. The structures were solved by SIR92/SIR97 [12] (direct methods) and refined using the SHELX-97 [13-14] software, both implemented in the program package WinGX22 [15]. Finally, all structures were checked using the PLATON software [16]. Structures displayed with ORTEP plots are drawn with thermal ellipsoids at 50 % probability level.

The theoretical calculations were achieved by using the GAUSSIAN16 program package [17] and were visualized by using GAUSSVIEW 6.0.16 [18]. Optimizations and frequency analyses were performed at the B3LYP level of theory (Becke's B3 three parameter hybrid functional by using the LYP correlation functional) with a cc-pVDZ basis set. After correcting the optimized structures with zero-point vibrational energies, the enthalpies and free energies were calculated on the CBS-4M (complete basis set) level of theory [19]. The detonation parameters were obtained by using the EXPLO5 (V6.05) program package [20-21].

### Example 1: N,N'-Bis(hydroxyethyl)oxamide

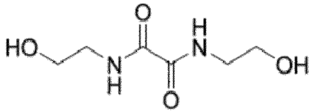

Diethyl oxalate (7.41 g, 50.7 mmol) was added dropwise to a stirred solution of solution of ethanolamine in toluene (100 mL). After stirring the solution for 24h at room temperature, the product precipitated and was filtered to recover N,N'-Bis(2-hydroxyethyl)oxamide as a white solid (8.54 g, 96%).

¹H NMR ([D₆]DMSO): *δ* = 8.56 (t, 2H, ³*J*(¹H,¹H) = 5.9 Hz, N*H*), 4.73 (t, 2H, ³*J*(¹H,¹H) = 5.6 Hz, O*H*), 3.47-3.42 (m, 4H, C*H*₂), 3.23-3.18 ppm (m, 4H, C*H*₂).

¹³C NMR ([D₆]DMSO): *δ* = 160.0 (*C*ONH), 59.2 (*C*H₂O), 41.7 ppm (*C*H₂).

EA: C₆H₁₂N₂O₄ (176.17 g mol⁻¹): calc. C 40.91, H 6.87, N 15.90 %; found C 40.89, H 6.78, N 15.89 %.

### Example 2: Bis(2-nitratoethyl) dinitroxamide

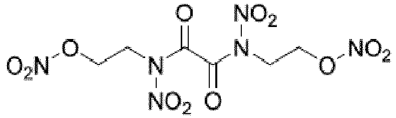

Nitric acid (100%, 10 mL) was added slowly to ice cold oleum (25%, 10 mL). N,N'-Bis(2-hydroxyethyl)oxamide (1.01 g, 5.75 mmol) was added in small portions under constant cooling with an ice bath. The reaction mixture was stirred for 1h at room temperature and afterwards poured onto 100 mL of ice whereby a colorless sticky solid precipitated. The mixture was extracted with ethyl acetate (3 × 50 mL). The combined organic phases were washed with water, until no more yellow coloring of the aqueous phase was observed. The organic phase was then washed with brine (50 mL) and dried over magnesium sulfate. The organic solvent was removed under reduced pressure to give N,N'-Bis(2-nitratoethyl)dinitroxamide as a colorless solid (1.44 g, 70%). ¹H NMR ([d₆]acetone): *δ* = 4.98 (t, 4H, C*H*₂O), 4.8 ppm (br, 4H, C*H*₂N)

¹³C NMR ([d₆]acetone): *δ* = 159.3 (*C*O), 69.5 (*C*H₂O), 44.4 ppm (*C*H₂N).

¹⁵N NMR ([d₆]acetone): *δ* = -44.1(t, ³*J*(¹⁵N,¹H) = 4.0 Hz, N*N*O₂), -45.7 (t, ³J(¹⁵N,¹H) = 3.1 Hz, O*N*O₂), -164.0 ppm (t, ²*J*(¹⁵N,¹H) = 3,5 Hz, *N*NO₂). The triplets observed are due to coupling to methylene hydrogen atoms.

Raman (1000 mW): *ν̃* = 3598 (8), 3062 (2), 3055 (6), 3027 (27), 3018 (4), 2987 (100), 2955 (2), 2682 (7), 2315 (9), 1743 (2), 1734 (4), 1724 (43), 1643 (10), 1600 (11), 1467 (13), 1433 (21), 1387 (8), 1362 (24), 1346 (5), 1299 (6), 1283 (76), 1265 (4), 1246 (28), 1227 (2), 1135 (8), 893 (7), 874 (2), 865 (15), 843 (12), 822 (47), 732 (11), 669 (10), 609 (4), 594 (11), 571 (44), 512 (23), 476 (13), 360 (12), 321 (10), 309 (5), 267 (7).

IR (ATR): *ν̃* = 3039 (vw), 2991 (vw), 2911 (vw), 1734 (w), 1722 (w), 1703 (m), 1639 (s), 1578 (s), 1463 (w), 1432 (m), 1425 (m), 1386 (w), 1372 (m), 1361 (w), 1346 (w), 1301 (m), 1274 (s), 1248 (s), 1225 (s), 1142 (w), 1131 (m), 1096 (m), 1041 (w), 1041 (w), 1006 (m), 987 (s), 969 (s), 940 (m).

EA: C₆H₈N₆O₁₂ (356.16 g mol⁻¹): calc. C 20.23, H 2.26, N 23.60 %; found C 20.35, H 2.13, N 23.67 %.

### Characterization of the crystal structure of bis(2-nitratoethyl) dinitroxamide

The crystal structure and data of bis(2-nitratoethyl) dinitroxamide were obtained as described in the section "Materials and Methods". Said data are collated in the table below.

**Table 2**

| formula | C₆H₈N₆O₁₂ |
|---|---|
| *MW*[g mol⁻¹] | 356.16 |
| *T*[K] | 173 |
| λ [Å] | 0.71073 |
| crystal system | monoclinic |
| space group | *P*2₁/*n* |
| crystal size [mm] | 0.18×0.15×0.10 |
| crystal habit | colorless block |
| *a* [Å] | 6.4950(4) |
| *b* [Å] | 13.8303(9) |
| *c* [Å] | 15.2696(11) |
| *α* [deg] | 90 |
| *β* [deg] | 100.538(2) |
| *γ* [deg] | 90 |
| *V* [Å³] | 1348.50(16) |
| *Z* | 4 |
| ρ_{calc.} [g cm⁻³] | 1.754 |
| *µ* [mm⁻¹] | 0.173 |
| F(000) | 728 |
| 2Θ range [deg] | 2.946 - 28.284 |
| index ranges | -8 ≤ *h* ≤ 8 |
| | -18 ≤ *k* ≤ 18 |
| | -20 ≤ *l* ≤20 |
| reflections collected | 32612 |
| reflections unique | 31790 |
| parameters | 249 |
| GooF | 1.063 |
| R₁/wR₂ [I>2σ(I)] | 0.0298 / 0.0729 |
| R₁/wR₂ (all data) | 0.0352 / 0.0765 |
| max / min residual electron density [Å⁻³] | -0.255 / 0.217 |
| CCDC | 2079484 |

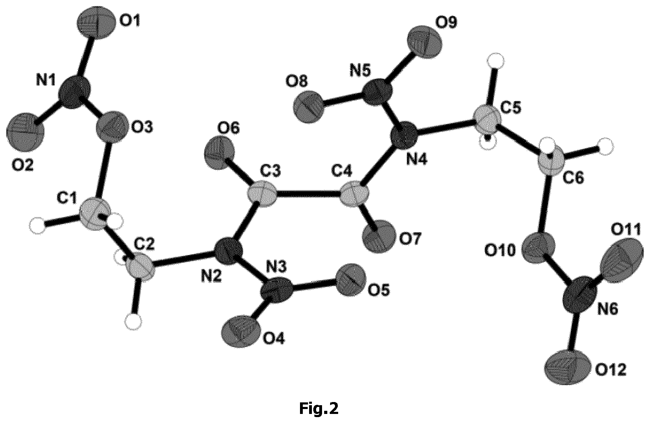

Bis(2-nitratoethyl) dinitroxamide crystallizes as colorless blocks in the monoclinic space group P2₁/*n* from dichloromethane. At 173 K (-100°C), the density is 1.754 g cm⁻³ and the structure with selected bonds and angles is shown in Figure 2.

Selected distances [Å] and angles [°] are as follows: C3-C4 1.5278(17), O3-C1 1.4535(17), N2-C3 1.3890(16), N2-N3 1.3935(15), O6-C3 1.2042(15), O3-N1 1.4070(15), O2-N1 1.2027(17), O6-C3-C4 118.07(11), O7-C4-C3 117.76(11), N2-C3-C4 120.19(11), O3-C1-C2 112.45(11), N3-N2-C2 118.46(10), O6-C3-C4-O7 -79.83(15), O6-C3-C4-N4 88.35(15), N3-N2-C3-O6 -170.90(11), C4-N4-N5-O9 179.83(11), C6-O10-N6-O12 179.17(12), N2-C3-C4-N4 -103.24(13).

### References

[1] T. M. Klapötke, Chemistry of High-Energy Materials, 5th ed., Walter de Gruyter, Berlin, 2019
[2] https://echa.europa.eu/substance-information/-/substanceinfo/100.028.647 (accessed 07.2021)
[3] H. F. R. Schoeyer, A. J. Schnorhk, P. A. O. G. Korting, P. J. van Lit, J. M. Mul, G. M. H. J. L. Gadiot, J. J. Meulenbrugge, J. Propul. Power 1995, 11, 856-869
[4] J. C. Bottaro, P. EI. Penwell, R. J. Schmitt, J. Am. Chem. Soc. 1997, 119, 9405-9410
[5] Q. J. Axthammer, T. M. Klapötke, B. Krumm, R. Moll, S. F. Rest, Z. Anorg. Allg. Chem. 2014, 640, 76-83
[6] Q. J. Axthammer, B. Krumm, T. M. Klapötke, J. Org. Chem. 2015, 80, 6329-6335
[7] T. M. Klapötke, B. Krumm, R. Scharf, Eur. J. Inorg. Chem. 2016, 3086-3093
[8] R. S. Stuart, G.F. Wright, Can. J. Res. 1948, 26B, 401-414
[9] Mendeleev Commun., 2013, 23, 81-83
[10] 1.171.35.11 ed., Oxford Diffraction Ltd., Abingdon, Oxford (U.K.), **2011**
[11] 1.171.35.11 ed., Oxford Diffraction Ltd., Abingdon, Oxford (U.K.), **2011**
[12] A. Altomare, M. C. Burla, M. Camalli, G. L. Cascarano, C. Giacovazzo, A. Gualiardi, A. G. G. Moliterni, G. Polidori, R. Spagna, J. Appl. Crystallogr. 1999, 32, 155-119
[13] G. M. Sheldrick, Programs for Crystal Structure Determination, University of Göttingen, Germany, 1997
[14] G. M. Sheldrick, Acta Crystallogr. 2008, 64A, 112-122
[15] L. Farrugia, J. Appl. Crystallogr. 1999, 32, 837-838
[16] A. L. Spek, Acta Crystallogr. 2009, 65D, 148-155
[17] http://gaussian.com/
[18] R. D. Dennington, T. A. Keith, J. M. Millam, GaussView, Ver. 6.0.16 ed., Semichem Inc., Shawnee Mission, KS, 2016
[19] J. A. Montgomery, M. J. Frisch, J. W. Ochterski, G. A. Petersson, J. Chem. Phys. 2000, 112, 6532-6542
[20] M. Sućeska, EXPLO5 V.6.03, Zagreb, 2015
[21] M. Sućeska, Propellants, Explos. Pyrotech. 1991, 16, 197-202

## Claims

1. A process for synthesizing bis(2-nitratoethyl)dinitroxamide which comprises reacting bis-hydroxyethyloxamide with nitric acid in the presence of an oleum solution.

2. The process of claim 1, wherein the oleum solution is a 20% to 65% oleum solution, preferably a 20% to 40% oleum solution, more preferably the oleum solution is a 25% or 30% oleum solution.

3. The process of claim 1 or claim 2, wherein from 5 to 15 equivalents of nitric acid are used for one equivalent of oleum solution.

4. The process of any preceding claim, wherein the nitric acid is fuming nitric acid, preferably nitric acid with a concentration > 98%.

5. The process of any preceding claim, wherein, the reaction of bis-hydroxyethyloxamide with nitric acid is carried out at a temperature in the range from about -5°C to about +10°C, preferably in the range from about -5°C to about +5°C.

6. The process of any preceding claim, wherein the reaction of bis-hydroxyethyloxamide with nitric acid is carried out for a period in the range from about 30 min to about 4h, preferably in the range from about 30 min to about 2h.

7. A process for preparing an energetic composition which comprises:
(i) preparing bis(2-nitratoethyl)dinitroxamide by the process as defined in any of claims 1 to 6;
(ii) admixing the resulting compound with at least one compound selected from an additional energetic material, an additive and a metal.

8. The process of claim 7, wherein at least one additional energetic material is admixed to bis(2-nitratoethyl)dinitroxamide in step (ii).

9. The process of claim 7 or claim 8, wherein the amount of energetic material(s) in the prepared energetic composition is at least 70 wt%, and up to 95 wt%, based on the weight of the energetic composition.
